Europäisches Patentamt

European Patent Office

Office européen des brevets

(11) Numéro de publication : **0 011 528**
**B1**

# (12) FASCICULE DE BREVET EUROPÉEN

(45) Date de publication du fascicule du brevet :
**20.02.85**

(21) Numéro de dépôt : **79400767.4**

(22) Date de dépôt : **19.10.79**

(51) Int. Cl.⁴ : **A 61 B 17/58, A 61 L 17/00**

(54) **Pièces d'ostéosynthèse et leur préparation.**

(30) Priorité : **20.10.78 FR 7829878**

(43) Date de publication de la demande :
**28.05.80 Bulletin 80/11**

(45) Mention de la délivrance du brevet :
**20.02.85 Bulletin 85/08**

(84) Etats contractants désignés :
**CH DE GB IT NL**

(56) Documents cités :
**FR-A- 2 364 644**
**FR-A- 2 391 734**
**US-A- 3 867 190**
**US-A- 3 982 543**

(73) Titulaire : **CENTRE NATIONAL DE LA RECHERCHE
SCIENTIFIQUE (C.N.R.S.)**
**15, Quai Anatole France**
**F-75007 Paris (FR)**

(72) Inventeur : **Vert, Michel**
**19, rue de Bargtehelde**
**F-76250 Deville-lès-Rouen (FR)**
Inventeur : **Chabot, François**
**3, rue du Framboisier**
**F-76000 Rouen (FR)**
Inventeur : **Leray, Jean**
**55, rue des Fontenelles**
**F-92310 Sèvres (FR)**
Inventeur : **Christel, Pascal**
**7, rue des Cloys**
**F-75018 Paris (FR)**

(74) Mandataire : **Nony, Michel**
**Conseil en Brevets d'Invention 29 rue Cambacérès**
**F-75008 Paris (FR)**

Jouve, 18, rue St-Denis, 75001 Paris, France

## Description

La présente invention a pour objet de nouvelles pièces d'ostéosynthèse, ainsi que leur préparation.

L'invention a plus particulièrement pour objet des pièces d'ostéosynthèse, notamment plaques, vis, clous, broches et fiches, réalisées avec un matériau plastique biocompatible et entièrement résorbable in vivo, qui se présentent sous la forme de pièces composites. Elles présentent des propriétés mécaniques suffisantes pour apporter une aide efficace et sûre au squelette traumatisé, pendant des temps suffisamment longs pour permettre la réparation et la consolidation de fractures y compris pour des os longs. Elles présentent également une bonne biorésorbabilité, suffisante pour permettre d'éviter une seconde intervention chirurgicale souvent nécessaire pour enlever les pièces d'ostéosynthèse lorsque celles-ci sont métalliques.

Les matériaux plastiques dérivés des polyesters d'acide α-hydroxy-acétique (ou acide glycolique) de hauts poids moléculaires ont été décrits dans le brevet US n° 2.668.162 et ceux dérivés des polyesters de l'acide lactique ont été décrits dans le brevet US n° 2.703.316.

Les matériaux à base de polyester d'acide α-hydroxy-acétique (PGA) ont été reconnus comme biocompatibles, biorésorbables et aptes à donner des sutures chirurgicales biorésorbables (brevet US n° 3.297.033).

L'utilisation des pièces d'ostéosynthèse faites de polyester d'acide hydroxy-acétique (PGA) a été décrite dans le brevet US n° 3.739.773, de même que le renforcement de ces pièces par fabrication de composites avec des éléments non absorbables par l'organisme.

Le brevet US n° 3.982.543 décrit un fil de suture en PGA renforcé à l'aide d'un revêtement obtenu par immersion d'un copolymère d'acide glycolique et d'acide lactique. Ce brevet ne suggère pas des pièces massives composites avec une matrice en polymère à base d'acide lactique, et des pièces de renforcement en PGA.

Le brevet français n° 2.364.644 décrit un matériau pour prothèse osseuse. Ce matériau non composite, en polymère biocompatible résorbable, contient une charge susceptible de favoriser la repousse osseuse.

La présente invention a pour objet des compositions pour utilisation en chirurgie osseuse comme pièces d'ostéosynthèse composites ayant la forme d'un solide tridimensionnel, à base de polymère biocompatible résorbable, caractérisées par le fait qu'elles sont constituées par une matrice en poly-acide lactique ou en un copolymère de celui-ci, ladite matrice contenant des éléments de renfort, noyés dans la matrice, constitués d'un homopolymère d'acide polyglycolique ou d'un copolymère de celui-ci, et contenant éventuellement une charge capable de stimuler la résorption du polymère au profit d'un tissu néoformé.

Les pièces d'ostéosynthèse de l'invention présentent encore les caractéristiques suivantes :
— Le poly-acide lactique constituant la matrice doit avoir une haute pureté optique de façon à posséder un degré de stéréo-régularité et de cristallinité élevé. On utilise soit un polymère de l'acide D-lactique, soit, de préférence, un polymère de l'acide L-lactique. La pureté énantiométrique doit être supérieure à 90 %.

On peut également utiliser des copolymères biodégradables de l'acide lactique et d'un comonomère compatible. Parmi ces comonomères, on peut citer des dérivés d'α-hydroxy-acides, des dérivés d'α-amino-acides, par exemple des carboxy-anhydrides d'α-amino-acides tels que ceux de l'alanine ou des lactones telles que la β-méthyl propiolactone. Les copolymères d'acide lactique utilisables englobent les stéréo-copolymères qui sont des copolymères des acides L- et D-lactiques. D'une façon générale, les copolymères d'acide lactique utilisés sont ceux qui contiennent suffisamment de motifs acide lactique pour posséder un degré de cristallinité élevé. Ces copolymères contiennent, en nombre de motifs, au moins 90 % et de préférence au moins 95 %, de motifs de dérivés de l'acide lactique afin de pouvoir conserver des propriétés mécaniques suffisantes pendant un temps approprié après mise en place de la pièce d'ostéosynthèse ;
— les éléments de renfort sont présents à raison de 5 à 50 %, en particulier de 10 à 40 % en poids, par rapport au poids total de la pièce d'ostéosynthèse ;

Les pièces d'ostéosynthèse de l'invention peuvent encore présenter les caractéristiques suivantes, prises isolément ou en combinaison :
— la matrice est obtenue au départ d'un homopolymère ou d'un copolymère d'acide lactique, de masse moléculaire élevée, tel que $\overline{M}p$ soit supérieure ou égale à 80 000 ou, de préférence, à 100 000 ;
— les éléments de renfort sont constitués par un homopolymère ou un copolymère d'acide glycolique, de masse moléculaire élevée tel que $\overline{M}p$ soit supérieure ou égale à 10 000, et généralement comprise entre 10 000 et 100 000. Les copolymères sont ceux obtenus avec des comonomères compatibles et biorésorbables, par exemple l'acide lactique ou le lactide, et ses formes optiquement actives. De préférence, les copolymères à base d'acide glycolique utilisés comme éléments de renfort contiennent en nombre de motifs, au moins 90 % de motifs dérivés de l'acide glycolique.

De tels polymères peuvent être préparés selon des procédés analogues à ceux décrits par exemple dans les brevets US n° 2.676.945 et 3.297.033 ;
— les éléments de renfort noyés dans la matrice ont la forme de fibres, de fils, de films, de tissus, de tresses ou de mats. Il est important que ces éléments soient bien noyés dans la matrice, sans apparaître à la surface de la pièce d'ostéosynthèse. Ils doivent donc être disposés à distance de cette surface ;

— lesdites pièces d'ostéosynthèse se présentent sous la forme de plaques, de clous ou de vis, ou sous forme de blocs solides à mouler ou à usiner ;

— lesdites pièces d'ostéosynthèse renferment une charge constituée de produits contenant l'un au moins des ions calcium, magnésium, sodium, potassium, phosphate, borate, carbonate ou silicate, ladite charge étant de préférence présente à raison de 0,5 à 5 % en poids par rapport au poids total de la pièce d'ostéosynthèse.

Comme indiqué ci-dessus en connaissait déjà des pièces d'ostéosynthèse à base de PGA.

Toutefois, le PGA se dégrade beaucoup trop vite après implantation dans l'organisme (d'après les expériences de la demanderesse, il est possible d'observer des signes très nets d'attaque au bout de 2 semaines). En conséquence le PGA ne peut pas assurer de manière absolue une aide sûre, c'est-à-dire garder d'excellentes propriétés mécaniques, et en particulier de résistance au choc pendant des temps suffisamment longs pour assurer la réparation des parties du squelette de mammifères lourds.

Le poly-acide lactique (PLA), en particulier le poly-acide L-lactique, constitué de polymères de hauts poids moléculaires, est un bon matériau plastique comparable aux meilleurs plastiques usuels, même en atmosphère normalement humide, à condition d'éliminer les produits de faible masse moléculaire et d'éviter des séjours prolongés à haute température. Dans ces conditions le PLA est un bon plastique, biocompatible et biorésorbable mais beaucoup plus lentement que le PGA. En particulier, il conserve une grande partie de ses propriétés mécaniques pendant au moins deux mois.

Il est moins cristallin que le PGA et a de ce fait une meilleure résistance au choc, comparable à celle des plastiques reconnus comme résilients.

Enfin le PLA a un point de fusion de 175 °C, donc nettement inférieur à celui du PGA (220 °C) ou des copolymères de celui-ci à faible teneur en comonomères (207 °C par exemple pour le copolymère à 10 % de lactide).

Par conséquent, en renforçant une matrice de PLA par des fils ou autres renforts en PGA, on tire le maximum des propriétés de chacun en évitant les inconvénients ; le PLA apporte une résilience de base suffisante et une bonne stabilité in vivo tandis que le PGA renforce la matrice sans être détérioré ou endommagé au moulage, ce qui est très important, et se trouve initialement protégé du milieu vivant ou il est facilement attaqué. En outre, la résorption de la matrice devient plus rapide après que les éléments de renfort initialement noyés dans la matrice sont devenus apparents.

On obtient ainsi :

— une matrice qui ne détériore pas le tissu ou les éléments (fibres, mat, etc.) de renfort biodégradable, ceux-ci ayant un point de fusion beaucoup plus élevé et, par conséquent, n'étant que faiblement dégradés thermiquement au moulage, ce qui assure l'efficacité du renfort ;

— des pièces composites qui conservent une forte proportion de leur résilience au choc même après deux mois d'implantation in vivo ;

— des pièces dont la résilience, de même que la résistance à la traction, est beaucoup plus élevée que celles des pièces non composites en PLA ou PGA ;

— des pièces qui peuvent être moulées ou usinées à partir d'éléments moulés au voisinage du point de fusion du poly-L-lactide (175 °C) par compression avec refroidissement rapide, ce qui limite considérablement la dégradation thermique qui fait apparaître de courtes chaînes dont les groupements terminaux favorisent la dégradation par hydrolyse tant à l'air qu'in vivo ;

— des pièces dont la matrice est constituée par un matériau renfermant peu de polymères de bas poids moléculaire, parce que ceux-ci sont éliminés physiquement avant la mise en œuvre et parce que l'on évite d'en former par dégradation au moulage ou que l'on en minimise la formation ;

— des pièces dont la résistance au choc est considérablement améliorée par le fait que le tissu de renfort est initialement protégé du milieu biologique, et par le fait que la matrice et le renfort adhèrent l'une à l'autre de façon satisfaisante par suite d'une préimprégnation ;

— des pièces stérilisables à l'oxyde d'éthylène à froid, ce qui constitue dans le cas présent la meilleure méthode, et notamment la moins dégradante, contrairement aux méthodes utilisant la chaleur ou les rayonnements ionisants ;

— des pièces manipulables à l'air ou même en atmosphère humide à température ordinaire sans précautions particulières pendant des durées classiques d'interventions chirurgicales.

L'intérêt de ces pièces renforcées est très grand notamment pour les plaques et clous d'ostéosynthèse qui travaillent en flexion et au choc.

L'invention a également pour objet un procédé de préparation d'une pièce d'ostéosynthèse telle que définie précédemment, caractérisé par le fait que l'on introduit par couches alternées, dans un moule à compression, le polymère constituant la matrice et les éléments de renfort, que l'on comprime l'ensemble à une pression suffisante, par exemple de 20 à 500 kg/cm² ($1,96 \times 10^6$ à $4,9 \times 10^7$ Pa) à température convenable pouvant généralement varier de 170 à 200 °C environ, pendant un temps de quelques minutes, de préférence inférieur à 7 min environ, et généralement compris entre 30 secondes et 7 minutes, pour lui donner la forme et les dimensions désirées, que l'on refroidit ensuite rapidement et que l'on démoule lorsque la température est inférieure à 50 °C.

Il est clair pour l'homme de l'art que les conditions du moulage dépendent de trois variables qu'il convient d'équilibrer : la pression, la température et le temps de chauffage. Dans le cas présent, il est important de limiter le temps de séjour à chaud des polymères, afin d'éviter ou de minimiser leur dégradation. Il convient en outre d'opérer à la température la plus basse possible, en tenant compte toutefois, bien entendu, des capacités de compression du moule utilisé.

Les exemples suivants illustrent l'invention sans toutefois la limiter :

Exemple 1

Préparation de poly-L-acide lactique

Le monomère L-lactide est purifié par recristallisation dans l'acétate d'éthyle puis dans l'acétone jusqu'à obtention d'un pouvoir rotatoire spécifique [α]58925 au moins égal à − 300° (solution dans le benzène).

Dans le réacteur de polymérisation, on introduit le monomère et le catalyseur (poudre de Zn à raison de 0,05 % en poids par rapport au monomère). On procède à un dégazage très soigné à l'aide d'une pompe à vide (1 à 2 mm de Hg) et en purgeant 3 fois par de l'azote ou de l'argon. Le réacteur est scellé sous vide puis placé à 140 °C pendant 20 jours en assurant une agitation tant que la viscosité du milieu le permet. Après refroidissement complet, on réduit la masse de polymère en copeaux de quelques dizièmes de mm d'épaisseur afin de procéder à une extraction solide-liquide par l'acétone pendant 24 heures. Le polymère, débarrassé des produits de faibles masses moléculaires est séché pendant 48 heures dans une étuve à vide à 50 °C. Finalement, il est conservé à l'abri de l'humidité.

Exemple 2

Moulage d'une pièce avec renfort de PGA

Le renfort biodégradable (tissu, mat, tresse...) est imprégné pendant 1 heure par une solution de chloroforme contenant 10 % de PLA. Puis il est séché sous vide à 40 °C pendant 24 heures afin d'éliminer le solvant et les traces d'humidité.

Dans un moule à compression préchauffé à 190 °C introduit par couches alternées le PLA (matrice) et les éléments de renfort. Les dimensions des éléments de renfort, ainsi que leur position dans la matrice sont choisies de telle sorte qu'ils soient complètement noyés dans la matrice. Puis on comprime lentement en faisant croître la pression jusqu'à 200 kg/cm² (1,96 × 10⁷ Pa) en une minute. On maintient cette pression pendant 2 minutes, la température étant toujours fixée à 190 °C. Finalement, on refroidit rapidement et on démoule lorsque la température est inférieure à 50 °C.

Selon ce mode opératoire, on a procédé au moulage d'une pièce renforcée par un tissu biodégradable. Le tissu est réalisé à partir de fils en acide polyglycolique d'un diamètre de 0,1 mm, commercialisé par Robert & Carrière sous la dénomination Ercedex ®.

La chaîne est constituée par des fils espacés de 1 mm tandis que la trame est constituée de fils jointifs.

Dans un moule à compression on introduit alternativement des lits de poudre de PLA, obtenu selon l'exemple 1, et des couches de tissu de renfort.

Après compression, on obtient un disque de 6 mm d'épaisseur dans lequel on découpe 2 éprouvettes entaillées IZOD.

On a mesuré la résilience du matériau ainsi obtenu, qui est égale à 90 kg/cm².

Exemple 3

Moulage d'une pièce chargée au phosphate tricalcique et renforcée par des fibres de PGA

Le phosphate tricalcique en poudre est séché pendant 24 heures à 60 °C. Dans un broyeur à billes on introduit le PLA obtenu selon l'exemple 1, puis le phosphate tricalcique, à raison de 1 % en poids par rapport au poids du PLA. On procède au broyage pendant 10 min puis le mélange obtenu est séché sous pression réduite pendant 24 heures à 40 °C.

Finalement, on réalise une pièce composite selon le procédé décrit à l'exemple 2, en utilisant comme polymère de matrice le PLA chargé avec le phosphate tricalcique. On a donné à la pièce la forme d'une plaque d'ostéosynthèse ayant les dimensions appropriées.

Exemple 4

Préparation de clous d'ostéosynthèse

Ces clous, de forme et de dimensions usuelles, sont préparés par moulage selon un procédé analogue à celui de l'exemple 2.

La matrice est constituée de PLA obtenu selon l'exemple 1. L'élément de renfort est constitué par un tissu de PGA en forme de tube. On dispose ledit tube de façon qu'il soit entièrement noyé dans le PLA de la matrice.

On a également réalisé des clous d'ostéosynthèse dans lesquels l'élément de renfort est constitué par des filaments de PGA disposés en spirale.

**Revendications**

1. Compositions pour utilisation en chirurgie osseuse comme pièces d'ostéosynthèse composites, ayant la forme d'un solide tridimensionnel, à base de polymère biocompatible résorbable, caractérisées par le fait qu'elles sont constituées par une matrice en poly-acide lactique ou en un copolymère de celui-ci, ledit copolymère comprenant, en nombre de motifs, au moins 90 % de motifs dérivés de l'acide lactique, ladite matrice contenant des éléments de renfort, noyés dans la matrice, constitués d'un homopolymère ou d'un copolymère d'acide polyglycolique, lesdits éléments de renfort ayant la forme de fils, de films, de tissus, de tresses ou de mats et étant présents à raison de 5 à 50 % en poids par rapport au poids total de la composition, et qu'elles contiennent éventuellement une charge capable de stimuler la résorption du polymère au profit d'un tissu néoformé.

2. Compositions selon la revendication 1, caractérisées par le fait que la matrice est constituée par un polymère de l'acide D-lactique, ou de l'acide L-lactique, de haute pureté énantiomérique.

3. Compositions selon la revendication 1, caractérisées par le fait que la matrice est constituée par un copolymère bio-résorbable de l'acide lactique et d'un comonomère compatible.

4. Compositions selon la revendication 3, caractérisées par le fait que ledit copolymère comprend 95 % de motifs dérivés de l'acide lactique.

5. Compositions selon l'une quelconque des revendications 3 et 4, caractérisées par le fait que ledit comonomère est choisi dans le groupe constitué par des dérivés d'$\alpha$-hydroxy-acides, des dérivés d'$\alpha$-amino-acides, notamment des carboxy-anhydrides d'$\alpha$-amino-acides tels que ceux de l'alanine, et des lactones telles que la $\beta$-méthyl propiolactone.

6. Compositions selon l'une quelconque des revendications précédentes, caractérisées par le fait que la matrice est obtenue au départ d'un poly-acide lactique ou d'un copolymère de celui-ci, de masse moléculaire élevée, telle que la masse moléculaire moyenne soit supérieure ou égale à 80 000.

7. Compositions selon la revendication 6, caractérisées par le fait que ladite masse moléculaire est supérieure ou égale à 100 000.

8. Compositions selon l'une quelconque des revendications précédentes, caractérisées par le fait que les éléments de renfort sont constitués par un polymère ayant une masse moléculaire supérieure ou égale à 10 000.

9. Compositions selon la revendication 8, caractérisées par le fait que ladite masse moléculaire est comprise entre 10 000 et 100 000.

10. Compositions selon l'une quelconque des revendications précédentes, caractérisées par le fait que les éléments de renfort sont réalisés en acide polyglycolique.

11. Compositions selon l'une quelconque des revendications 1 à 9, caractérisées par le fait que les éléments de renfort sont constitués en un copolymère de l'acide glycolique avec un comonomère compatible et biorésorbable.

12. Compositions selon la revendication 11, caractérisées par le fait que ledit comonomère est choisi dans le groupe constitué par l'acide lactique ou le lactide, et ses formes optiquement actives.

13. Compositions selon l'une quelconque des revendications 11 et 12, caractérisées par le fait que ledit copolymère à base d'acide glycolique utilisé comme élément de renfort contient, en nombre de motifs, au moins 90 % de motifs dérivés de l'acide glycolique.

14. Compositions selon l'une quelconque des revendications précédentes, caractérisées par le fait qu'elles se présentent sous la forme de plaques, de clous ou de vis, ou sous forme de blocs solides à mouler ou à usiner.

15. Compositions selon l'une quelconque des revendications précédentes, caractérisées par le fait qu'elle renferment une charge constituée de produits contenant l'un au moins des ions calcium, magnésium, sodium, potassium, phosphate, borate, carbonate et silicate.

16. Compositions selon la revendication 15, caractérisées par le fait que la charge est présente à raison de 0,5 à 5 % en poids, par rapport au poids total de la pièce d'ostéosynthèse.

17. Procédé de préparation d'une composition selon l'une quelconque des revendications précédentes, caractérisé par le fait que l'on introduit par couches alternées, dans un moule à compression, le polymère constituant la matrice et les éléments de renfort, que l'on comprime l'ensemble à une pression et température convanables pendant un temps de quelques minutes, pour lui donner la forme et les dimensions désirées, que l'on refroidit ensuite rapidement et que l'on démoule lorsque la température est inférieure à 50 °C.

18. Procédé selon la revendication 17, caractérisé par le fait que ladite pression peut varier de 20 à 500 kg/cm$^2$ (1,96 × 10$^6$ à 4,9 × 10$^7$ Pa).

19. Procédé selon l'une quelconque des revendications 17 et 18, caractérisé par le fait que ladite température peut varier de 170 à 200 °C.

20. Procédé selon l'une quelconque des revendications 17 à 19, caractérisé par le fait que le temps de compression est inférieur à 7 minutes et généralement compris entre 30 secondes et 7 minutes.

**Claims**

1. Compositions for use in bone surgery as composite osteosynthesis pieces, having the shape of a three-dimensional solid, the compositions being based on a biocompatible absorbable polymer, characterised in that they consist of a matrix of poly(lactic acid) or of a copolymer thereof, the said copolymer comprising, in terms of number of units, at least 90 % of the units derived from lactic acid, and the said matrix containing enforcing elements embedded in the matrix and consisting of a homopolymer or copolymer of polyglycolic acid, the said reinforcing elements having the shape of filaments, films, fabrics, braids or mats and being present in an amount of 5 to 50 % by weight relative to the total weight of the composition, and in that they optionally contain a filler capable of stimulating the absorption of the polymer to the benefit of newly formed tissue.

2. Compositions according to Claim 1, characterised in that the matrix consists of a polymer of D-lactic acid or of L-lactic acid, of high enantiometric purity.

3. Compositions according to Claim 1, characterised in that the matrix consists of a bio-absorbable copolymer of lactic acid and a compatible comonomer.

4. Compositions according to Claim 3, characterised in that the said copolymer contains 95 % of units derived from lactic acid.

5. Compositions according to any one of Claims 3 and 4, characterised in that the said comonomer is chosen from the group consisting of derivatives of α-hydroxyacids, derivatives of α-aminoacids, especially of carboxy-anhydrides of α-aminoacids such as those of alanine, and lactones such as β-methyl-propiolactone.

6. Compositions according to any one of the preceding claims, characterised in that the matrix is botained from a poly(lactic acid) or a copolymer of lactic acid, of high molecular weight, such that the mean molecular mass is greater than or equal to 80,000.

7. Compositions according to Claim 6, characterised in that the said molecular mass is greater than or equal to 100,000.

8. Compositions according to any one of the preceding claims, characterised in that the reinforcing elements consist of a polymer having a molecular mass greater than or equal to 10,000.

9. Compositions according to Claim 8, characterised in that the said molecular mass is between 10,000 and 100,000.

10. Compositions according to any one of the preceding claims, characterised in that the reinforcing elements are made of polyglycolic acid.

11. Compositions according to any one of Claims 1 to 9, characterised in that the reinforcing elements consist of a copolymer of glycolic acid with a compatible and bioabsorbable comonomer.

12. Compositions according to Claim 11, characterised in that the said comonomer is chosen from the group consisting of lactic acid or lactide and its optically active forms.

13. Compositions according to either of Claims 11 and 12, characterised in that the said copolymer based on glycolic acid and used as a reinforcing element contains, in terms of number of units, at least 90 % of the units derived from glycolic acid.

14. Compositions according to any one of the preceding claims, characterised in that they are in the form of plates, nails or screws or in the form of solid blocks which can be moulded or machined.

15. Compositions according to any one of the preceding claims, characterised in that they contain a filler consisting of products containing at least one of the following ions : calcium, magnesium, sodium, potassium, phosphate, borate, carbonate and silicate.

16. Compositions according to Claim 15, characterised in that the filler is present in an amount of 0.5 to 5 % by weight relative to the total weight of the osteosynthesis piece.

17. Process for the preparation of a composition according to any one of the preceding claims, characterised in that the polymer constituting the matrix and the reinforcing elements are introduced in alternate layers into a compression mould, the whole is compressed under a suitable pressure and at a suitable temperature for a period of several minutes to give it the desired shape and dimensions, that thereafter it is cooled rapidly and that the piece is demoulded when the temperature is below 50 °C.

18. Process according to Claim 17, characterised in that the said pressure can vary between 20 and 500 kg/cm$^2$ (1.96 × 10$^6$ to 4.9 × 10$^7$ Pa).

19. Process according to any one of Claims 17 and 18, characterised in that the said temperature can vary from 170 to 200 °C.

20. Process according to any one of Claims 17 to 19, characterised in that the compression time is less than 7 minutes and is generally between 30 seconds and 7 minutes.

## Ansprüche

1. Zusammensetzung zur Verwendung in der Knochenchirurgie, wie zusammengesetzte Stücke für den Knochenaufbau in Form einer dreidimensionalen festen Substanz auf der Basis eines biokompatiblen resorbierbaren Polymeren, dadurch gekennzeichnet, daß sie aus einer Matrix aus Polymilchsäure oder eines Copolymeren davon besteht, wobei dies Copolymer eine Anzahl Molekülfolgen enthält, von denen mindestens 90 % von Milchsäure abgeleitet sind, und wobei die Matrix Verstärkungselemente enthält, die in der Matrix versenkt sind und aus einem Homopolymerisat oder einem Copolymerisat der Polyglykolsäure bestehen und wobei diese Verstärkungselemente in Form von Fäden, Filmen, Geweben, Geflechten oder Matten vorliegen und in einer Menge von 5 bis 50 Gewichtsprozent, bezogen auf das Gesamtgewicht der Zusammensetzung, vorhanden sind und die gegebenenfalls eine Ladung aufweisen, die in der Lage ist, die Resorption des Polymerisats zugunsten eines neugebildeten Gewebes zu stimulieren.

2. Zusammensetzung nach Anspruch 1, dadurch gekennzeichnet, daß die Matrix aus einem Polymeren der D-Milchsäure oder der L-Milchsäure großer enantiomerer Reinheit besteht.

3. Zusammensetzung nach Anspruch 1, dadurch gekennzeichnet, daß die Matrix aus einem bioresorbierbaren Copolymerisat von Milchsäure und einem damit verträglichen Comonomeren besteht.

4. Zusammensetzung nach Anspruch 3, dadurch gekennzeichnet, daß das Copolymerisat 95 % Moleküle enthält, die von Milchsäure abgeleitet sind.

5. Zusammensetzung nach einem der Ansprüche 3 und 4, dadurch gekennzeichnet, daß das Comonomer ausgewählt ist aus der Gruppe bestehend aus Derivaten der α-Hydroxysäuren, Derivaten der α-Aminosäuren, nämlich Carboxyanhydriden der α-Aminosäuren, wie solches des Alanins, und der Lactone, wie β-Methylpropiolacton.

6. Zusammensetzung nach den vorhergehenden Ansprüchen, dadurch gekennzeichnet, daß die Matrix erhalten worden ist ausgehend von einer Polymilchsäure oder eines Copolymerisats davon mit höherem Molekulargewicht als das mittlere Molekulargewicht, das größer oder gleich 80 000 ist.

7. Zusammensetzung nach Anspruch 6, dadurch gekennzeichnet, daß das Molekulargewicht größer oder gleich 100 000 ist.

8. Zusammensetzung nach den vorhergehenden Ansprüchen, dadurch gekennzeichnet, daß die Verstärkungselemente aus einem Polymerisat mit einem Molekulargewicht größer oder gleich 10 000 bestehen.

9. Zusammensetzung nach Anspruch 8, dadurch gekennzeichnet, daß das Molekulargewicht zwischen 10 000 und 100 000 beträgt.

10. Zusammensetzung nach einem der vorhergehenden Ansprüche, dadurch gekennzeichnet, daß die Verstärkungselemente aus Polyglykolsäure bestehen.

11. Zusammensetzung nach einem der Ansprüche 1 bis 9, dadurch gekennzeichnet, daß die Verstärkungselemente aus einem Copolymerisat von Glykolsäure mit einem damit verträglichen und bioresorbierbaren Comonomer bestehen.

12. Zusammensetzung nach Anspruch 11, dadurch gekennzeichnet, daß das Comonomer ausgewählt ist aus der Gruppe bestehend aus Milchsäure oder einem Lactid und den optisch aktiven Formen davon.

13. Zusammensetzung nach einem der Ansprüche 11 und 12, dadurch gekennzeichnet, daß das Copolymerisat auf der Basis von Glykolsäure, das als Verstärkungselement verwendet wird, mindestens 90 % Moleküle, die von Glykolsäure abgeleitet sind, in der Molekülfolgenzahl enthält.

14. Zusammensetzung nach den vorhergehenden Ansprüchen, dadurch gekennzeichnet, daß sie in der Form von Platten, Nägeln oder Schrauben oder in Form fester Blöcke zum Gießen oder zum maschinellen Verformen vorliegt.

15. Zusammensetzung nach den vorhergehenden Ansprüchen, dadurch gekennzeichnet, daß sie eine Ladung einschließt, die aus einem Produkt besteht, das mindestens eines der Ionen Calcium, Magnesium, Natrium, Kalium, Phosphat, Borat, Carbonat und Silikat enthält.

16. Zusammensetzung nach Anspruch 15, dadurch gekennzeichnet, daß die Ladung vorliegt in einer Menge von 0,5 bis 5 Gewichtsprozent, bezogen auf das Gesamtgewicht des für den Knochenaufbau verwendeten Teils.

17. Verfahren zum Herstellen einer Zusammensetzung nach den vorhergehenden Ansprüchen, dadurch gekennzeichnet, daß man in abwechselnden Schichten das Polymerisat, das die Matrix und die Verstärkungselemente bildet, in eine Druckgießform einbringt, und die zusammengebrachten Polymerisate bei einem Druck und bei einer Temperatur über einige Minuten soweit komprimiert, daß das Formteil in den gewünschten Dimensionen erhalten wird, und daß man anschließend schnell abkühlt und entformt, wenn die Temperatur unter 50 °C beträgt.

18. Verfahren nach Anspruch 17, dadurch gekennzeichnet, daß der Druck von 20 bis 500 kg/cm$^2$ (1,96 × 10$^6$ bis 4,9 × 10$^7$ Pa) schwanken kann.

19. Verfahren nach einem der Ansprüche 17 und 18, dadurch gekennzeichnet, daß die Temperatur von 170 bis 200 °C schwanken kann.

20. Verfahren nach einem der Ansprüche 17 bis 19, dadurch gekennzeichnet, daß die Kompressionsdauer unter 7 Minuten und allgemein zwischen 30 Sekunden und 7 Minuten beträgt.